# EUROPÄISCHE PATENTSCHRIFT

(11) **EP 0 645 158 B1**
(45) Veröffentlichungstag und Bekanntmachung des Hinweises auf die Patenterteilung: **09.06.1999**
(21) Anmeldenummer: 94110950.6
(22) Anmeldetag: 14.07.1994
(51) Int. Cl.: A61M 25/01

(54) **Abwinkelbarer Führungskatheter für medizinische Zwecke**
Bendable guide catheter for medical purposes
Cathéter-guide pliable à usage médical

(30) Priorität: 25.09.1993 DE 4332667
(43) Veröffentlichungstag der Anmeldung: 29.03.1995
(73) Patentinhaber: Aesculap AG & Co. KG, 78532 Tuttlingen (DE)
(72) Erfinder: Wieneke, Paul, D-78604 Rietheim-Weilheim (DE); Nesper, Markus, D-78532 Tuttlingen (DE)
(74) Vertreter: Hoeger, Stellrecht & Partner

(56) Entgegenhaltungen:
- EP-A- 0 306 010
- EP-A- 0 358 117
- EP-A- 0 401 129
- US-A- 3 773 034
- US-A- 5 179 934

## Beschreibung

Die Erfindung betrifft einen abwinkelbaren Führungskatheter für medizinische Zwecke mit einem Arbeitskanal und einem schlauchförmigen Mantel, der eine sich in Längsrichtung des Mantels erstreckende Kammer zur Aufnahme eines fließfähigen Mediums bildet, wobei der Mantel durch Füllung der Kammer mit dem Medium elastisch dehnbar ist, und mit einer Wicklung aus im wesentlichen nicht dehnbarem Material, die, bezogen auf die Längsrichtung des Mantels, in Höhe der Kammer schraubenlinienförmig mit definiertem Steigungswinkel um den Mantel gewickelt ist.

Führungskatheter werden dazu verwendet, medizinische Instrumente, wie zum Beispiel Scheren, Pinzetten oder auch Lichtleitfasern, zu einem entsprechenden Behandlungs- oder Untersuchungsgebiet in sehr engen Körperhöhlen zu führen. Dazu werden die Führungskatheter in eine Körperöffnung eingeführt, wobei sie einen Arbeitskanal bilden. Die medizinischen Instrumente werden entweder in einem weiteren Arbeitsschritt reversibel in den Arbeitskanal eingeschoben und nach Gebrauch wieder entfernt, oder die medizinischen Instrumente, beispielsweise Lichtleitfasern, sind im Arbeitskanal fest verankert, so daß sie gleichzeitig mit dem Führungskatheter in die Körperöffnung eingeführt werden. Um das gezielte Erreichen eines bestimmten Behandlungs- oder Untersuchungsgebiets zu gewährleisten, beispielsweise das Abbiegen in einer bestimmten Richtung am Ende eines engen Kanals, sind die Führungskatheter abwinkelbar ausgebildet und können vom Bedienungspersonal mit Hilfe von Steuerelementen abgelenkt werden.

Aus der US-PS 3,773,034 ist ein Katheter bekannt, der hydraulisch in verschiedene Richtungen abgewinkelt werden kann. Er umfaßt einen im Bereich des distalen Endes des Katheters in Längsrichtung dehnbaren, schlauchförmigen Mantel sowie ein Armierungselement, das exzentrisch und eine Längsdehnung des Mantels im Kontaktbereich zwischen dem Armierungselement und dem Mantel verhindernd mit dem Mantel verbunden ist. Außerdem umfaßt der Katheter eine sich in Längsrichtung des Mantels erstreckende ringförmige Kammer, die mit einem fliepfähigen Medium gefüllt werden kann und dadurch insbesondere in dem dem Armierungselement diametral entgegengesetzten Bereich des Mantels eine Längsdehnung hervorruft. Durch die einseitige Längsdehnung erfährt der Katheter insgesamt eine Abwinklung. Um zu verhindern, daß sich der Mantel in radialer Richtung ausdehnt, wenn die Kammer mit dem Medium gefüllt wird, ist der Mantel von einer Wicklung aus nicht dehnbarem Material umgeben. Da außerdem verhindert werden soll, daß sich der Katheter bei Füllung der Kammer auf seiner gesamten Länge abwinkelt, besteht der Mantel nur im Bereich des distalen Endes des Katheters aus in Längsrichtung dehnbarem Material, während sich der restliche Bereich des Mantels bei Füllung der Kammer im wesentlichen nicht ausdehnt.

Um zu ermöglichen, daß sich der in der US-PS 3,773,034 beschriebene Katheter nur in einem definierten Bereich abwinkelt, ist somit ein komplizierter konstruktiver Aufbau des Katheters notwendig. Der Katheter weist außerdem den Nachteil auf, daß er lediglich eine gleichmäßige Abwinklung mit nur einem Krümmungsradius ermöglicht. Beispielsweise ist es nicht möglich, durch Füllung der Kammer den unmittelbar an das distale Ende des Katheters angrenzenden Bereich stärker abzuwinkeln als einen vom distalen Ende weiter entfernt liegenden Bereich des Katheters.

Aus der EP 237 564 B1 ist ein Führungskatheter bekannt, der mechanisch abgewinkelt werden kann. Er umfaßt ein Verankerungs- und ein Betätigungselement sowie ein flexibles Element, das einseitig mit einem eine Längsdehnung verhindernden Armierungselement verbunden ist. Das flexible Element ist an seinem proximalen Ende mit dem Verankerungselement und an seinem distalen Ende mit dem Betätigungselement fest verbunden. Wird das Betätigungselement in proximale Richtung verschoben, so wird das flexible Element in Richtung auf das Verankerungselement kontrahiert und aufgrund der einseitigen Armierung abgewinkelt. Der in der EP 237 564 B1 vorgeschlagene Führungskatheter weist ebenfalls einen komplizierten konstruktiven Aufbau auf und ermöglicht ebenso wie der in der US-PS 3,773,034 beschriebene Katheter nur eine Abwinklung mit längs des flexiblen Elements konstantem Krümmungsradius. Der in der EP 237 564 B1 dargestellte Führungskatheter weist außerdem den Nachteil auf, daß mit der Abwinklung des Katheters eine radiale Ausdehnung des flexiblen Elements verbunden ist, die zu Verletzungen des den Führungskatheter umgebenden Körpergewebes führen kann.

Aufgabe der Erfindung ist es, ausgehend von der US-PS 3,773,034 einen gattungsgemäßen abwinkelbaren Führungskatheter für medizinische Zwecke so auszugestalten, daß er einen einfachen konstruktiven Aufbau aufweist und eine Abwinklung ermöglicht, bei der in verschiedenen Längsbereichen des Führungskatheters unterschiedliche Krümmungsradien erzielbar sind.

Diese Aufgabe wird bei einem abwinkelbaren Führungskatheter der eingangs beschriebenen Art erfindungsgemäß dadurch gelöst, daß die einzelnen Windungen der Wicklung in einem Teilwinkelbereich des Mantelumfangs in axialer Richtung relativ zueinander weniger verschiebbar sind als in einem weiteren Winkelbereich des Mantelumfangs und daß die Wicklung in Längsrichtung des Mantels Bereiche mit unterschiedlichem Steigungswinkel aufweist.

Die Erfindung basiert auf der Erkenntnis, daß die mechanische Stabilität einer schraubenlinienförmigen Wicklung gegenüber einer radial nach außen wirkenden Druckbelastung abhängig ist vom jeweiligen Steigungswinkel der Windungen. Unabhängig vom verwendeten Material hat eine derartige Wicklung bei einem Steigungswinkel von ungefähr 35° eine maximale mechanische Stabilität. Sofern die einzelnen Windungen der Wicklung in axialer Richtung relativ zueinander verschieblich gehalten sind, verschieben sich die einzelnen Windungen bei Druckbelastung der Wicklung dergestalt, daß sie sich mit zunehmendem Druck dem für die mechanische Stabilität optimalen Steigungswinkel von ungefähr 35° annähern. Haben die Windungen einmal diesen Steigungswinkel eingenommen, so wird dieser Steigungswinkel und damit der relative axiale Abstand der einzelnen Windungen zueinander auch bei weiterer Druckerhöhung beibehalten. Je größer die Differenz ist zwischen dem anfänglichen Steigungswinkel der Wicklung und dem für die mechanische Stabilität optimalen Steigungswinkel von ungefähr 35°, desto größer ist die Verschiebung der einzelnen Windungen relativ zueinander bei zunehmender Druckbelastung.

Bei dem erfindungsgemäßen Führungskatheter umgibt die schraubenlinienförmig angeordnete Wicklung einen Mantel, der auf die Wicklung einen radial nach außen wirkenden Druck ausübt, sofern eine vom Mantel gebildete Kammer mit einem fließfähigen Medium gefüllt wird. Da die einzelnen Windungen in einem ersten Teilwinkelbereich des Mantelumfanges relativ zueinander weniger verschiebbar sind als in einem zweiten Teilwinkelbereich des Mantelumfangs, verschieben sich die Windungen bei der durch die Füllung der Kammer hervorgerufenen Druckbelastung in dem zweiten Teilwinkelbereich des Mantelumfangs stärker relativ zueinander, um sich dem für die mechanische Stabilität der Wicklung optimalen Steigungswinkel von ungefähr 35° anzunähern, als im ersten Teilwinkelbereich, und rufen durch diese in Umfangsrichtung ungleichmäßige Verschiebung ihrer Lage eine Abwinklung des Führungskatheters hervor. Da die Wicklung vor Füllung der Kammer in Längsrichtung des Mantels Bereiche mit unterschiedlichem Steigungswinkel aufweist, bewirkt die durch die Füllung der Kammer hervorgerufene zunehmende Druckbelastung der Wicklung in verschiedenen Längsbereichen des Mantels eine unterschiedlich starke Verschiebung der einzelnen Windungen der Wicklung, bis diese jeweils den Steigungswinkel von ungefähr 35° eingenommen haben, so daß dadurch längs des Mantels eine unterschiedlich starke Abwinklung des Führungskatheters und somit verschiedene Krümmungsradien erzielt werden.

Da die ungleichmäßige Verschiebung der Windungen jeweils nur so lange andauert, bis diese einen Steigungswinkel von ungefähr 35° eingenommen haben, wird durch die erfindungsgemäße Ausgestaltung des Führungskatheters eine Begrenzung der Abwinklung, das heißt ein Winkelanschlag, erreicht. Je mehr der Steigungswinkel der Windungen ohne Druckbelastung der Kammer vom Wert von ungefähr 35° differiert, desto größer ist die erreichbare Abwinklung bis der Winkelanschlag erreicht wird und auch bei weiterer Verstärkung der Druckbelastung keine zusätzliche Abwinklung mehr erfolgt.

Wird die Druckbelastung anschließend wieder reduziert, so wirkt die Elastizität des Mantels als Rückstellkraft und der Führungskatheter nimmt wieder seine ursprüngliche Ausrichtung ein.

Bei einer in konstruktiver Hinsicht besonders vorteilhaften Ausgestaltung der Erfindung ist vorgesehen, daß sich die Kammer und die Wicklung im wesentlichen über die gesamte Länge des Mantels erstrecken. Da der Grad der Abwinklung des Führungskatheters von der Differenz des Steigungswinkels der Windungen ohne Druckbelastung zu dem für die mechanische Stabilität optimalen Steigungswinkel von ungefähr 35° abhängt, ist es nicht erforderlich, die Kammer und die Wicklung auf einen Längsbereich des Führungskatheters zu beschränken, in dem eine Abwinklung beabsichtigt ist, sondern die Kammer und die Wicklung können sich über die gesamte Länge des Mantels erstrecken, wodurch ein besonders kostengünstiges Herstellungsverfahren ermöglicht wird.

Vorteilhaft ist es, in Längsbereichen des Führungskatheters, die bei Füllung der Kammer keine Abwinklung erfahren sollen, eine Wicklung mit einem Steigungswinkel von ungefähr 35° zu verwenden. Dadurch entfällt der Gebrauch zusätzlicher Mittel, durch die eine Abwinklung vermieden werden soll, beispielsweise die Benutzung von den Mantel umgebenden Hülsen und dergleichen, da sich die einzelnen Windungen bei einem anfänglichen Steigungswinkel von ungefähr 35° bei Druckbelastung in ihrem axialen Abstand zueinander nicht verändern und somit keine Abwinklung hergerufen wird. Da die Windungen bei einem Steigungswinkel von ungefähr 35° jeder Veränderung ihres axialen Abstands zueinander entgegenwirken, wird in Bereichen mit einem Steigungswinkel von ungefähr 35° durch die Füllung der Kammer lediglich bewirkt, daß der Führungskatheter seine Flexibilität in diesem Bereich verliert und in seiner ursprünglichen Orientierung erstarrt.

Günstig ist es, in einem dem proximalen Ende des Führungskatheters benachbarten Bereich der Wicklung einen Steigungswinkel von ungefähr 35° zu verwenden, da sich dadurch dieser Bereich des Führungskatheters bei Füllung der Kammer nicht abwinkelt und somit eine gute Handhabbarkeit des Führungskatheters erzielt wird.

Bei einer vorteilhaften Ausführungsform der Erfindung ist vorgesehen, daß die Wicklung einen Bereich mit einem Steigungswinkel größer als 35° aufweist. Bei einem derartigen Steigungswinkel verschieben sich die einzelnen Windungen der Wicklung bei Druckbelastung in Richtung auf einen kleineren Steigungswinkel bis minimal ungefähr 35°, wodurch aufgrund der in Umfangsrichtung ungleichmäßigen Verschiebbarkeit der Windungen zueinander bewirkt wird, daß sich der Führungskatheter in Richtung des dem Bereich mit geringerer Verschiebbarkeit der einzelnen Windungen diametral entgegengesetzten Bereichs des Mantelumfanges abwinkelt.

Bei einer besonderen Ausführungsform des erfindungsgemäßen Führungskatheters ist vorgesehen, daß die Wicklung einen Bereich mit einem Steigungswinkel kleiner als 35° aufweist. In diesem Fall verschieben sich die einzelnen Windungen der Wicklung bei Druckbelastung in Richtung auf einen größeren Steigungswinkel, der maximal ungefähr 35° beträgt, und es wird eine Abwinklung des Führungskatheters in Richtung des Bereichs mit geringerer Verschiebbarkeit der Windungen hervorgerufen.

Vorteilhaft ist es, wenn ein Bereich der Wicklung mit einem Steigungswinkel ungleich ungefähr 35° dem distalen Ende des Führungskatheters benachbart angeordnet ist. Dadurch wird gewährleistet, daß der Führungskatheter im Bereich des distalen Endes abwinkelbar ist und sich von einer Bedienungsperson besonders einfach durch einen gewundenen, nichtlinearen Kanal führen läßt.

Um das distale Ende des Führungskatheters besonders stark abwinkelbar auszugestalten, ist in einer vorteilhaften Ausführungsform vorgesehen, daß sich der Steigungswinkel der Wicklung ausgehend von einem dem distalen Ende des Führungskatheters benachbarten Bereich in Richtung auf das proximale Ende des Führungskatheters mit zunehmender Entfernung vom distalen Ende einem Wert von ungefähr 35° annähert. Somit ist die Differenz des Steigungswinkels vom für die mechanische Stabilität der Wicklung optimalen Steigungswinkel von ungefähr 35° am distalen Ende des Führungskatheters besonders groß, wodurch in diesem Bereich eine besonders starke Abwinklung, das heißt ein besonders kleiner Krümmungsradius, erzielbar ist.

Um in einem Längsbereich des Führungskatheters eine stetige Änderung des Krümmungsradius zu erzielen, ist in einer weiteren vorteilhaften Ausführungsform vorgesehen, daß die Wicklung einen Bereich mit einem sich kontinuierlich ändernden Steigungswinkel unfaßt.

Nähert sich der Betrag des Steigungswinkels ausgehend vom distalen Ende des Führungskatheters in Richtung auf das proximale Ende gleichmäßig auf den für die mechanische Stabilität der Wicklung optimalen Wert von ungefähr 35°, so wird dadurch bei Füllung der Kammer eine Abwinklung des Führungskatheters in Form einer Spirale mit in Richtung auf das distale Ende gleichmäßig abnehmendem Krümmungsradius hervorgerufen.

Bei einer besonders vorteilhaften Ausführungsform des Führungskatheters ist vorgesehen, daß die Wicklung in einem Teilwinkelbereich des Mantelumfangs unverschieblich an einer Außenseite des Mantels gehalten ist. Dies ermöglicht mit einem konstruktiv besonders einfachen Aufbau des Führungskatheters die für die Abwinklung erforderliche relativ geringe Verschiebbarkeit der einzelnen Windungen der Wicklung in einem Teilwinkelbereich des Mantelumfangs.

Während die vorstehend beschriebene Ausführungsform eine konstruktiv besonders einfache und somit billige Lösung darstellt, ist bei einem etwas aufwendigeren, aber von der Funktion her verbesserten Führungskatheter vorgesehen, daß dieser ein sich in Längsrichtung des Mantels erstreckendes Armierungselement umfaßt, an dem die Wicklung in einem Teilwinkelbereich des Mantelumfangs unverschieblich gehalten ist. Die in Umfangsrichtung ungleichmäßige Verschiebbarkeit der einzelnen Wicklungen zueinander läßt sich dadurch unabhängig vom verwendeten Mantel erzielen. Dadurch ist es möglich, bei verschiedenen Ausführungsformen des Führungskatheters, die sich im jeweiligen Mantelmaterial unterscheiden, jeweils die gleichen Mittel zur ungleichmäßigen Verschiebbarkeit der Windungen zu verwenden.

Bei einer weiteren vorteilhaften Ausführungsform ist vorgesehen, daß das Armierungselement durch eine Armierungsfaser gebildet ist. Da diese besonders dünn ausgestaltet werden kann, ist für die ungleichmäßige Verschiebbarkeit der Wicklungswindungen nur ein geringer Platzbedarf notwendig und der Führungskatheter kann insgesamt besonders dünn ausgebildet werden.

Um zu erreichen, daß die Abwinklung des Führungskatheters in einer definierten Ebene erfolgt, ist in einer vorteilhaften Ausführungsform der Erfindung vorgesehen, daß die Armierungsfaser parallel zur Längsachse des Mantels an der Außenseite des Mantels positioniert ist. Während die Armierungsfaser prinzipiell in verschiedenen Längsbereichen des Mantels in Umfangsrichtung unterschiedliche Positionierungen einnehmen kann, wodurch eine Abwinklung des Führungskatheters nicht nur in einer Ebene sondern im gesamten Raum ermöglicht wird, ist es für die Handhabbarkeit des Führungskatheters günstig, wenn die Armierungsfaser parallel zur Längsachse des Mantels positioniert ist und somit die Abwinklung des Führungskatheters nur in der durch die Armierungsfaser und die Längsachse des Mantels definierten Ebene erfolgt.

Bei einer weiteren vorteilhaften Ausführungsform der Erfindung ist vorgesehen, daß der Führungskatheter zwei schraubenförmig den Mantel umgebende Wicklungen mit entgegengesetztem Drehsinn umfaßt. Die beiden Wicklungen weisen jeweils denselben Steigungswinkel auf und bewirken bei Füllung der Kammer aufgrund der jeweiligen Verschiebung ihrer Windungen eine besonders zuverlässige Abwinklung des Führungskatheters und dadurch eine besonders zuverlässige Funktionsfähigkeit.

Die folgende Beschreibung einer bevorzugten Ausführungsform der Erfindung dient im Zusammenhang mit der Zeichnung der näheren Erläuterung. Es zeigen:
- Figur 1:: eine schaubildliche Ansicht eines Längsabschnitts eines abwinkelbaren Führungskatheters mit zwei schraubenlinienförmigen Wicklungen mit entgegengesetztem Drehsinn, die jeweils einen Steigungswinkel von ungefähr 35° aufweisen;
- Figur 2:: eine Seitenansicht des in Figur 1 gezeigten Längsabschnitts;
- Figur 3:: eine Seitenansicht eines zweiten Längsabschnitts ähnlich Figur 2 in einem unbelasteten Zustand, wobei der Steigungswinkel der Wicklungen kleiner als ungefähr 35° ist;
- Figur 4:: eine Seitenansicht des in Figur 3 gezeigten Längsabschnitts im belasteten Zustand;
- Figur 5:: eine Seitenansicht eines dritten Längsabschnitts ähnlich Figur 2 im unbelasteten Zustand, wobei der Steigungswinkel der Wicklungen größer als ungefähr 35° ist;
- Figur 6:: eine Seitenansicht des in Figur 5 gezeigten Längsabschnitts in belastetem Zustand.

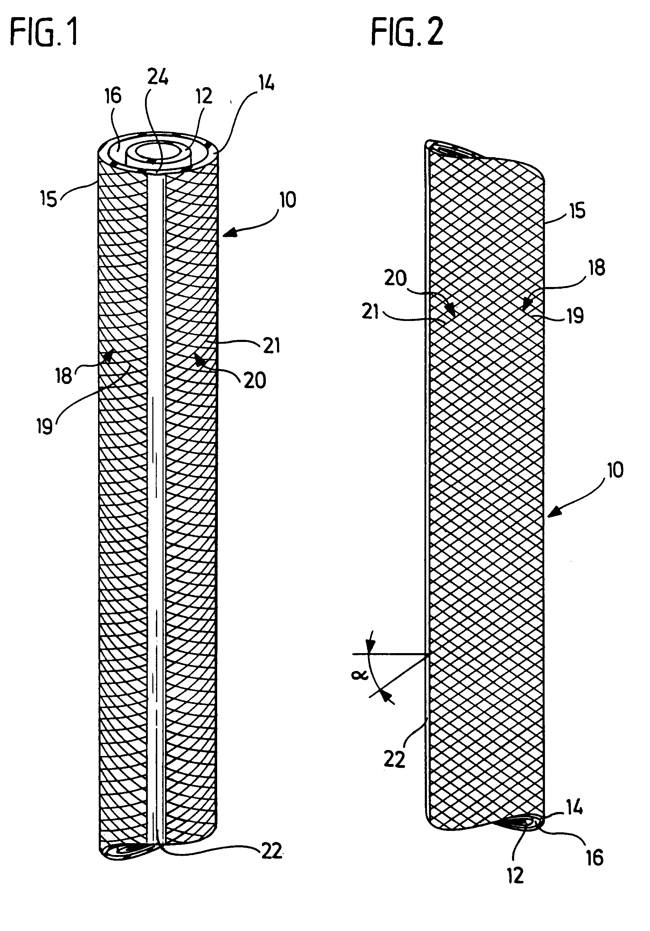

Figur 1 zeigt einen Längsabschnitt eines Führungskatheters mit einem doppelwandigen Mantel 10, der eine Innenwand 12 und eine Außenwand 14 umfaßt. Zwischen der Innenwand 12 und der Außenwand 14 erstreckt sich längs des Mantels 10 eine Kammer 16 mit ringförmigem Querschnitt, die, dies ist in der Zeichnung nicht dargestellt, am distalen Ende des Mantels abgeschlossen ist und die, dies ist in der Zeichnung ebenfalls nicht dargestellt, am proximalen Ende des Mantels über eine Fülleitung mit einem fließfähigen Medium auffüllbar ist.

Der Mantel 10 ist aus einem elastisch dehnbaren Material hergestellt. Die Außenwand 14 ist umgeben von zwei Wicklungen 18 und 20, die aus schraubenlinienförmig angeordneten Windungen 19 beziehungsweise 21 bestehen. Die Wicklung 18 weist dabei den entgegengesetzten Drehsinn der Wicklung 20 auf. Parallel zur Längsachse des Mantels 10 ist an einer Außenseite 15 der Außenwand 14 entlang der gesamten Länge des Mantels 10 eine Armierungsfaser 22 positioniert, wobei der Durchmesser der Armierungsfaser 22 beträchtlich kleiner ist als der Durchmesser der Außenwand 14, so daß die Armierungsfaser 22 lediglich einen Teilbereich 24 der Außenwand 14 überdeckt. Die Windungen 19 und 21 der beiden Wicklungen 18 und 20 sind im Teilbereich 24 an der Armierungsfaser 22 fixiert, im gesamten von der Armierungsfaser 22 nicht überdeckten Bereich der Außenseite 15 jedoch lediglich verschieblich an der Außenwand 14 gehalten.

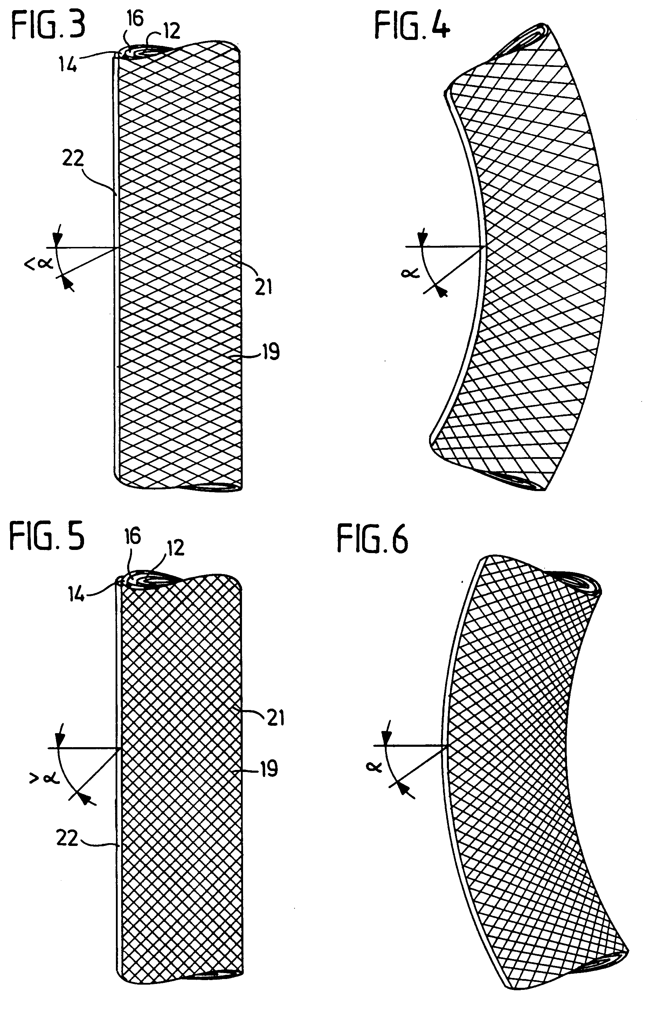

Wie aus Figur 2 deutlich wird, unterscheiden sich die beiden Wicklungen 18 und 20 zwar in ihrem Drehsinn, die einzelnen Windungen 19 beziehungsweise 21 weisen aber denselben Steigungswinkel α auf. Dabei wird der Steigungswinkel α definiert durch die schräg zur Längsachse des Mantels verlaufende Wicklungsrichtung und eine quer zur Längsrichtung positionierte Ebene. Bei dem in Figur 2 dargestellten Längsabschnitt des Führungskatheters beträgt der Steigungswinkel α ungefähr 35°. In Figur 2 werden wie in den weiteren Figuren 3 bis 6 für gleiche Bauteile dieselben Bezugszeichen verwendet wie in Figur 1.

Wird die Kammer 16 des Mantels 10 über die in der Zeichnung nicht dargestellte Fülleitung mit dem fließfähigen Medium gefüllt, so dehnt sich dadurch die Außenwand 14 des Mantels 10 radial nach außen aus und übt einen radial nach außen wirkenden Druck auf die Wicklungen 18 und 20 aus. Diese sind aus einem im wesentlichen nicht dehnbaren Material hergestellt, so daß die Windungen 19 und 21 auf die zunehmende Druckbelastung in dem nicht von der Armierungsfaser 22 überdeckten Bereich der Außenseite 15 der Außenwand 14 im allgemeinen mit einer Verschiebung in axialer Richtung, das heißt in Längsrichtung des Mantels, reagieren, um so ihren Abstand in axialer Richtung zu verändern und sich dem für die mechanische Stabilität der Wicklungen optimalen Steigungswinkel von ungefähr 35° anzunähern.

Da die Windungen 19 und 21 in dem in Figur 2 dargestellten Längsabschnitt des Führungskatheters bereits ohne Druckbelastung einen Steigungswinkel von ungefähr 35° aufweisen, verändern die Windungen auch bei zunehmender Druckbelastung ihren axialen Abstand zueinander nicht, so daß es in diesem Längsbereich des Führungskatheters auch bei Füllung der Kammer 16 zu keiner Abwinklung kommt. Die Füllung der Kammer 16 bewirkt in diesem Längsbereich lediglich, daß der Führungskatheter erstarrt und seine ursprüngliche geradlinige Ausrichtung beibehält.

In Figur 3 ist ein zweiter Längsabschnitt des Führungskatheters dargestellt, bei dem die Windungen 19 und 21 jeweils einen Steigungswinkel α aufweisen, der kleiner ist als der für die mechanische Stabilität der Wicklungen optimale Steigungswinkel von ungefähr 35°. Die durch die Füllung der Kammer 16 hervorgerufene Druckbelastung der Wicklungen 18 und 20 hat in dem in Figur 3 dargestellten Längsbereich des Führungskatheters eine Abwinklung in Richtung der einseitig positionierten Armierungsfaser 22 zur Folge. Dies ist in Figur 4 dargestellt. Die Abwinklung ergibt sich aus der Verschiebung der Windungen 19 und 21 in dem nicht von der Armierungsfaser 22 überdeckten Bereich der Außenseite 15. Die Windungen 19 und 21 verschieben sich dabei so lange, bis sie einen Steigungswinkel von ungefähr 35° erreichen. Anschließend verharren die Windungen 19 und 21 auch bei weiter zunehmender Druckbelastung in dieser Stellung, so daß dadurch ein Winkelanschlag gebildet wird, der einen minimalen Krümmungsradius definiert. Die maximal erreichbare Abwinklung des Führungskatheters und damit verbunden der minimale Krümmungsradius ist abhängig von der Differenz des Steigungswinkels α der Windungen 19 und 21 ohne Druckbelastung zu dem für die mechanische Stabilität der Wicklungen 18 und 20 optimalen Steigungswinkel von ungefähr 35°. Je größer die Differenz der Steigungswinkel ist, desto größer ist die maximal erreichbare Abwinklung des Führungskatheters und desto kleiner ist der erreichbare Krümmungsradius.

In Figur 5 ist ein dritter Längsabschnitt des Führungskatheters dargestellt, bei dem die entsprechenden Windungen 19 und 21 ohne Druckbelastung eine Steigungswinkel α einnehmen, der größer ist als der für die mechanische Stabilität optimale Steigungswinkel von ungefähr 35°. In diesem Fall verschieben sich die Windungen 19 und 21 in dem nicht von der Armierungsfaser 22 überdeckten Bereich der Außenseite 15 der Außenwand 14 dergestalt, daß sie sich mit zunehmender Druckbelastung dem Steigungswinkel von ungefähr 35° annähern und dadurch eine Abwinklung des Führungskatheters in die der einseitig angeordneten Armierungsfaser 22 diametral entgegengesetzte Richtung hervorrufen. Dies ist in Figur 6 dargestellt. Nachdem sich die Windungen 19 und 21 so lange an der Außenseite 15 verschoben haben, bis sie einen Steigungswinkel von ungefähr 35° eingenommen haben, bewirkt eine weitere Druckerhöhung keine weitere Verschiebung der Windungen mehr und damit auch keine weitere Abwinklung des Führungskatheters.

Wird die Kammer 16 des Mantels 10 wieder entleert, so verringert sich dadurch die Druckbelastung, und die Elastizität des Mantels 10 sowie des Geflechts der Armierungsfaser bewirkt, daß wieder die ursprüngliche Ausrichtung des Führungskatheters eingenommen wird.

Durch beliebiges Aneinanderreihen von Längsabschnitten, wie sie in den Figuren 2, 3 und 5 dargestellt sind, läßt sich somit insgesamt ein Führungskatheter erzielen, der eine Abwinklung ermöglicht, bei der verschiedene Längsbereiche unterschiedliche Krümmungsradien aufweisen, die durch die Wahl des Steigungswinkels α der Windungen 19 und 21 im unbelasteten Zustand definiert sind, und die nicht unterschritten werden können.

## Patentansprüche

1. Abwinkelbarer Führungskatheter für medizinische Zwecke mit einem Arbeitskanal und einem schlauchförmigen Mantel (10), der eine sich in Längsrichtung des Mantels erstreckende Kammer zur Aufnahme eines fließfähigen Mediums bildet, wobei der Mantel (10) durch Füllung der Kammer mit dem fließfähigen Medium elastisch dehnbar ist, und mit einer Wicklung (18; 20) aus im wesentlichen nicht dehnbarem Material, die, bezogen auf die Längsrichtung des Mantels (10), in Höhe der Kammer schraubenlinienförmig mit definiertem Steigungswinkel um den Mantel (10) gewickelt ist,
wobei die einzelnen Windungen (19; 21) der Wicklung (18; 20) in einem Teilwinkelbereich (24) des Mantelumfangs relativ zueinander in axiale Richtung weniger verschiebbar sind als in einem weiteren Winkelbereich des Mantelumfangs, dadurch gekennzeichnet daß die Wicklung (18; 20) in Längsrichtung des Mantels (10) Bereiche mit unterschiedlichem Steigungswinkel (α) aufweist.

2. Abwinkelbarer Führungskatheter nach Anspruch 1, dadurch gekennzeichnet, daß sich die Kammer (16) und die Wicklung (18; 20) im wesentlichen über die gesamte Länge des Mantels (10) erstrecken.

3. Abwinkelbarer Führungskatheter nach Anspruch 1 oder 2, dadurch gekennzeichnet, daß in einem der Bereiche die Wicklung (18; 20) einen Steigungswinkel (α) von ungefähr 35° aufweist.

4. Abwinkelbarer Führungskatheter nach Anspruch 3, dadurch gekennzeichnet, daß in einem der Bereiche die Wicklung (18; 20) mit einem Steigungswinkel (α) von ungefähr 35° dem proximalen Ende des Führungskatheters benachbart angeordnet ist.

5. Abwinkelbarer Führungskatheter nach einem der Ansprüche 1 bis 4, dadurch gekennzeichnet, daß in einem der Bereiche die Wicklung (18; 20) einen Steigungswinkel (α) größer als ungefähr 35° aufweist.

6. Abwinkelbarer Führungskatheter nach einem der voranstehenden Ansprüche, dadurch gekennzeichnet, daß in einem der Bereiche die Wicklung (18; 20) einen Steigungswinkel (α) kleiner als ungefähr 35° aufweist.

7. Abwinkelbarer Führungskatheter nach einem der voranstehenden Ansprüche, dadurch gekennzeichnet, daß ein Bereich der Wicklung (18; 20) mit einem Steigungswinkel (α) ungleich ungefähr 35° dem distalen Ende des Führungskatheters benachbart angeordnet ist.

8. Abwinkelbarer Führungskatheter nach Anspruch 7, dadurch gekennzeichnet, daß sich der Steigungswinkel (α) der Wicklung (18; 20) ausgehend von einem dem distalen Ende des Führungskatheters benachbarten Bereich in Richtung auf das proximale Ende des Führungskatheters mit zunehmender Entfernung vom distalen Ende einem Wert von ungefähr 35° annähert.

9. Abwinkelbarer Führungskatheter nach einem der voranstehenden Ansprüche, dadurch gekennzeichnet, daß die Wicklung (18; 20) einen Bereich mit einem sich kontinuierlich ändernden Steigungswinkel (α) aufweist.

10. Abwinkelbarer Führungskatheter nach einem der voranstehenden Ansprüche, dadurch gekennzeichnet, daß die Wicklung (18; 20) in dem Teilwinkelbereich (24) des Mantelumfangs unverschieblich an einer Außenseite (15) des Mantels (10) gehalten ist.

11. Abwinkelbarer Führungskatheter nach einem der voranstehenden Ansprüche, dadurch gekennzeichnet, daß der Führungskatheter ein sich in Längsrichtung des Mantels (10) erstreckendes Armierungselement (22) umfaßt, an dem die Wicklung (18; 20) in dem Teilwinkelbereich (24) des Mantelumfangs unverschieblich gehalten ist.

12. Abwinkelbarer Führungskatheter nach Anspruch 11, dadurch gekennzeichnet, daß das Armierungselement durch eine Armierungsfaser (22) gebildet ist.

13. Abwinkelbarer Führungskatheter nach Anspruch 12, dadurch gekennzeichnet, daß die Armierungsfaser (22) parallel zu einer Längsachse des Mantels (10) an der Außenseite (15) des Mantels (10) positioniert ist.

14. Abwinkelbarer Führungskatheter nach einem der voranstehenden Ansprüche, dadurch gekennzeichnet, daß der Führungskatheter eine zweite Wicklung aufweist, wobei die Wicklungen zwei schraubenlinienförmig den Mantel (10) umgebende Wicklungen (18, 20) mit entgegengesetztem Drehsinn darstellen.

## Claims

1. A bendable guide catheter for medical purposes, having a working channel and a tubular casing (10) which forms a chamber, extending in the longitudinal direction of the casing, to receive a flowable medium, the casing (10) being resiliently stretchable through filling the chamber with the flowable medium, and having a coil (18; 20) which is composed of substantially non-stretchable material and which, relative to the longitudinal direction of the casing (10), is along the extent of the chamber helically coiled around the casing (10) with a defined angle of inclination, the individual turns (19; 21) of the coil (18; 20) being displaceable relative to one another in the axial direction to a lesser extent in a partial angular region (24) of the casing circumference than in another angular region of the casing circumference, characterised in that in the longitudinal direction of the casing (10), the coil (18; 20) has regions whose angles of inclination (α) differ.

2. A bendable guide catheter in accordance with Claim 1, characterised in that the chamber (16) and the coil (18; 20) extend over substantially the entire length of the casing (10).

3. A bendable guide catheter in accordance with Claim 1 or 2, characterised in that in one of the regions, the coil (18; 20) has an angle of inclination (α) of approximately 35°.

4. A bendable guide catheter in accordance with Claim 3, characterised in that in one of the regions, the coil (18; 20) is arranged adjacent to the proximal end of the guide catheter with an angle of inclination (α) of approximately 35°.

5. A bendable guide catheter in accordance with any one of Claims 1 to 4, characterised in that in one of the regions, the coil (18; 20) has an angle of inclination (α) greater than approximately 35°.

6. A bendable guide catheter in accordance with any one of the preceding Claims, characterised in that in one of the regions, the coil (18; 20) has an angle of inclination (α) smaller than approximately 35°.

7. A bendable guide catheter in accordance with any one of the preceding Claims, characterised in that one region of the coil (18; 20) is arranged adjacent to the distal end of the guide catheter with an angle of inclination (α) which is not equal to approximately 35°.

8. A bendable guide catheter in accordance with Claim 7, characterised in that in the direction of the proximal end of the guide catheter, starting from a region adjacent to the distal end of the guide catheter, the angle of inclination (α) of the coil (18; 20) approaches a value of approximately 35° as the distance from the distal end increases.

9. A bendable guide catheter in accordance with any of the preceding Claims, characterised in that the coil (18; 20) has a region with a continually altering angle of inclination (α).

10. A bendable guide catheter in accordance with any one of the preceding Claims, characterised in that in the partial angular region (24) of the casing circumference, the coil (18; 20) is held on an outer surface (15) of the casing (10) in a non-displaceable manner.

11. A bendable guide catheter in accordance with any one of the preceding Claims, characterised in that the guide catheter comprises a reinforcement element (22) which extends in the longitudinal direction of the casing (10) and on which the coil (18; 20) in the partial angular region (24) of the casing circumference is held so as to not be displaceable.

12. A bendable guide catheter in accordance with Claim 11, characterised in that the reinforcement element is formed by a reinforcement fibre (22).

13. A bendable guide catheter in accordance with Claim 12, characterised in that the reinforcement fibre (22) is positioned on the outer surface (15) of the casing (10) so as to be parallel to a longitudinal axis of the casing (10).

14. A bendable guide catheter in accordance with any one of the preceding Claims, characterised in that the guide catheter has a second coil, the coils constituting two coils (18, 20) which helically surround the casing (10) and which are coiled in opposite directions.

## Revendications

1. Cathéter de guidage pliable à usage médical comportant un canal de travail et une enveloppe en forme de tuyau (10) qui forme une chambre qui s'étend dans la direction longitudinale de l'enveloppe pour recevoir un milieu fluide, où l'enveloppe (10,) est extensible élastiquement par remplissage de la chambre avec le milieu fluide, et un enroulement (18 ; 20) en un matériau sensiblement non extensible qui, par rapport à la direction longitudinale de l'enveloppe (10), est enroulé autour de l'enveloppe (10) à la hauteur de la chambre en forme d'hélice avec un angle d'hélice défini, où les différentes spires (19 ; 21) de l'enroulement ( 18 ; 20) sont moins déplaçables les unes par rapport aux autres en direction axiale dans un domaine angulaire partiel (24) de la périphérie de l'enveloppe que dans un autre domaine angulaire de la périphérie de l'enveloppe, caractérisé en ce que l'enroulement (18 ; 20) comporte dans la direction longitudinale de l'enveloppe (10) des domaines ayant un angle d'hélice (α) différent.

2. Cathéter de guidage pliable selon la revendication 1, caractérisé en ce que la chambre (16) et l'enroulement (18 ; 20) s'étendent sensiblement sur toute la longueur de l'enveloppe (10).

3. Cathéter de guidage pliable selon la revendication 1 ou 2, caractérisé en ce que, dans l'un des domaines, l'enroulement (18 ; 20) présente un angle d'hélice (α) d'environ 35°.

4. Cathéter de guidage pliable selon la revendication 3, caractérisé en ce que, dans l'un des domaines, l'enroulement (18 ; 20) est agencé au voisinage de l'extrémité proximale du cathéter de guidage avec un angle d'hélice (α) d'environ 35°.

5. Cathéter de guidage pliable selon l'une des revendications 1 à 4, caractérisé en ce que, dans l'un des domaines, l'enroulement (18 ; 20) présente un angle d'hélice (α) supérieur à environ 35°.

6. Cathéter de guidage pliable selon l'une des revendications précédentes, caractérisé en ce que, dans l'un des domaines, l'enroulement (18 ; 20) présente un angle d'hélice (α) inférieur à environ 35°.

7. Cathéter de guidage pliable selon l'une des revendications précédentes, caractérisé en ce qu'un domaine de l'enroulement (18 ; 20) ayant un angle d'hélice (α) différent d'environ 35° est disposé au voisinage de l'extrémité distale du cathéter de guidage.

8. Cathéter de guidage pliable selon la revendication 7, caractérisé en ce que l'angle d'hélice (α) de l'enroulement (18 ; 20) tend vers une valeur d'environ 35° depuis un domaine voisin de l'extrémité distale du cathéter de guidage en direction de l'extrémité proximale du cathéter de guidage, en s'éloignant de plus en plus de l'extrémité distale.

9. Cathéter de guidage pliable selon l'une des revendications précédentes, caractérisé en ce que l'enroulement (18 ; 20) présente un domaine ayant un angle d'hélice (α) qui varie de manière continue.

10. Cathéter de guidage pliable selon l'une des revendications précédentes, caractérisé en ce que l'enroulement (18 ; 20) est maintenu immobile au niveau d'un côté externe (15) de l'enveloppe (10) dans le domaine angulaire partiel (24) de la périphérie de l'enveloppe.

11. Cathéter de guidage pliable selon l'une des revendications précédentes, caractérisé en ce que le cathéter de guidage comporte un élément d'armature (22) qui s'étend dans la direction longitudinale de l'enveloppe (10) et au niveau duquel est maintenu non déplaçable l'enroulement (18 ; 20) dans le domaine angulaire partiel (24) de la périphérie de l'enveloppe.

12. Cathéter de guidage pliable selon la revendication 11, caractérisé en ce que l'élément d'armature est formé par une fibre d'armature (22).

13. Cathéter de guidage pliable selon la revendication 12, caractérisé en ce que la fibre d'armature (22) est positionnée parallèlement à un axe longitudinal de l'enveloppe (10) au niveau du côté externe (15) de l'enveloppe (10).

14. Cathéter de guidage pliable selon l'une des revendications précédentes, caractérisé en ce que le cathéter de guidage comporte un deuxième enroulement où les enroulements constituent deux enroulements (18, 20) de sens de rotation opposés qui entourent en hélice l'enveloppe (10).
